# EUROPEAN PATENT SPECIFICATION

(11) **EP 0 739 420 B1**
(45) Date of publication and mention of the grant of the patent: **02.05.2002**
(21) Application number: 95905106.1
(22) Date of filing: 22.12.1994
(51) Int. Cl.: C12Q 1/68, C12N 9/18, C07K 14/33

(54) **PRIMERS FOR THE AMPLIFICATION OF GENES CODING FOR THE ENTEROTOXIN AND THE LECITHINASE OF CLOSTRIDIUM PERFRINGENS AND THEIR APPLICATION TO THE DETECTION AND COUNTING OF THESE BACTERIAE**
PRIMERS ZUR GENAMPLIFIKATION VON ENTEROTOXIN UND LECITHINASE AUS CLOSTRIDIUM PERFRINGENS UND DEREN ANWENDUNG ZUR NACHWEIS UND ZÄHLUNG DIESER BAKTERIEN
SONDES POUR L'AMPLIFICATION DE GENES CODANT POUR L'ENTEROTOXINE ET LA LICITHINASE DE CLOSTRIDIUM PERFRINGENS AINSI QUE LEURS APPLICATIONS DANS LA DETECTION ET LA NUMERATION DE CES BACTERIES

(30) Priority: 22.12.1993 US 172026
(43) Date of publication of application: 30.10.1996
(73) Proprietor: INSTITUT PASTEUR, 75724 Paris Cédex 15 (FR); Agence Française de Sécurité Sanitaire des Aliments, 94701 Maisons-Alfort (FR)
(72) Inventor: FACH, Patrick, F-94000 Créteil (FR); GUILLOU, Jean-Pierre, F-94430 Chennevières (FR); POPOFF, Michel, F-92140 Clamart (FR)
(74) Representative: Phélip, Bruno
(86) International application number: PCT/EP94/04292
(87) International publication number: WO 95/17521

(56) References cited:
- EP-A- 0 395 292
- EP-A- 0 409 159
- J APPL BACTERIOL, (1993 JAN) 74 (1) 61-6., FACH P; GUILLOU J P 'Detection by in vitro amplification of the alpha-toxin ( phospholipase C) gene from Clostridium perfringens .'
- MED. MAL. INFECT., (1993) 23/2 (70-77)., FACH P.; DELBART M.O.; SCHLACHTER A.; POUMEYROL M.; POPOFF M.R. 'APPORTS DE LA TECHNIQUE D'AMPLIFICATION GENIQUE (PCR) AU DIAGNOSTIC DES TOXI-INFECTIONS ALIMENTAIRES A CLOSTRIDIUM PERFRINGENS'
- INT. J. FOOD MICROBIOL., vol. 17, no. 1, September 1992 NL, pages 47-55, SAITO ET AL. 'Detection of C. perfringens enterotoxin gene by the PCR procedure'
- INFECTION AND IMMUNITY, vol. 57, no. 2, WASHINGTON US, pages 367-376, TITBALL ET AL. 'Molecular cloning ...' cited in the application
- BIOCHEM. BIOPHYS. RES. COMMUN. 160:33-39(1989), OKABE A., SHIMIZU T., HAYASHI H. 'Cloning and sequencing of a phospholipase C gene of Clostridium perfringens'
- MOL. MICROBIOL. 3:383-392(1989)., LESLIE D., FAIRWEATHER N., PICKARD D., DOUGAN G., KEHOE M. 'Phospholipase C and hemolytic activities of clostridium perfringens alpha-toxin, cloned in Escherichia coli: sequence and homology with a Bacillus cereus phospholipase C"'
- ANTONIE VAN LEEUWENHOEK, vol. 56, 1989 NL, pages 181-190, VAN DAMME-JONGSTEN ET AL. 'Cloning and sequencing of the C. perfringens enterotoxin gene' cited in the application
- INFECTION AND IMMUNITY, vol. 61, no. 8, August 1993 US, pages 3429-3439, CZECZULIN ET AL. 'Cloning, nucleotide sequencing and expression of the C. perfringens enterotoxin gene in E. coli'
- J APPL BACTERIOL, (1994 DEC) 77 (6) 650-5., DAUBE G; CHINA B; SIMON P; HVALA K; MAINIL J 'Typing of Clostridium perfringens by in vitro amplification of toxin genes.'

## Description

The present invention relates to primers for the amplification of genes coding for the enterotoxin and the lecithinase , also called phospholipase C, of Clostridium perfringens.

Another object of the invention is the application of these primers for the detection and the numeration of C. perfringens.

Clostridium perfringens type A is widely distributed (soil, sewage, intestinal tracts of humans and animals), and is a common causative agent of bacterial food poisoning outbreaks worldwide. The symptoms, predominantly diarrhea and abdominal pain, appear 6 to 24 hours after ingestion of contaminated food. Vomiting and fever are unusual. Death occurs occasionally among debilitated persons, particularly the elderly.

C. perfringens enterotoxin CPE which is produced during the sporulation phase has been shown to produce the symptoms associated with C. perfringens food poisoning. The illness is caused by the ingestion of food that contains larger number of vegetative enterotoxigenic C. perfringens ( more than 10⁵ organisms per g) . These bacteriae multiply and sporulate, releasing CPE into the intestine.

A C. perfringens count of more than 10⁶/g in fecal samples of ill persons is indicative of C. perfringens food poisoning . In addition, CPE detection directly in fecal samples is a valuable method confirming the diagnosis.

The epidemiological investigations involve C. perfringens numeration in suspected foods. The characterization of the enterotoxigenic C. perfringens strains is not used routinely, since C. perfringens sporulates poorly in usual culture media.

Recently, CPE and phospholipase C gene sequences have been determined (VAN DAMME et al. 1989, Ant. Van Leeuwen 56, 181-190; TSO J. and SIEBEL, 1989. Infect. Immun. 57: 468-476, TITBALL et al. 1989 Infect. Immun. 57:367-376). The phospholipase C gene is located on a variable region of the chromosomal DNA in all C. perfringens toxinotypes whereas the distribution of CPE gene is restricted. Only 6% of the C. perfringens isolates from various origins showed the presence of CPE gene by DNA-DNA hybridization. This ratio is higher (59%) among C. perfringens strains isolated from confirmed outbreaks of food poisoning. In the standard methods, the enterotoxin detection by biological or immunological tests requires previously the sporulation of C. perfringens. Several specific medium and protocols for C. perfringens sporulation have been described, but they are time consuming and many C. perfringens strains do not sporulate or very poorly (DUCAN and STRONG, 1968. Appl. Microbiol. 16: 82; PHILIPS , 1986 , Lett. Appl. Microbiol 3: 77-79), which impairs the CPE detection.

A method for the detection of C. perfringens by polymerase chain reaction (PCR) has yet been disclosed during the third Congress of the French Society of Microbiology ( April 21-24, 1992) through a poster of FACH et al.

The method was consisting in an amplification of the parts of the genes encoding the α-toxin, also called lecithinase, and the enterotoxin of C. perfringens by using oligonucleotidic primers which were choosen in these genes. However, the sequence of the primers used for carrying out this method was not disclosed and the sensitivity mentioned by the authors was low , i.e. from 500 to 5000 bacteriae per gram of feces.

Moreover, the results were obtained on feces artificially contaminated and not on feces from contaminated patients or on contaminated food.

The inventors have thus sought to elaborate a sensitive and reliable method allowing the detection of low quantities of bacteriae in samples of different origins, such as in feces or food, in a raw form.

They have surprisingly shown that it is necessary to choose the primers in some well determined regions of the genes, as well as in the gene of the enterotoxin that in the one of the lecithinase.

Besides , they have carried out a process for the treatment of foods samples, allowing a specific, sensitive and reliable determination of the presence of the C. perfringens contained in these foods.

Primers for the amplification of the gene encoding the lecithinase of C. perfringens, also called alphatoxin or phospholipase C, corresponding to a part of the sequence of the gene comprised between the nucleotides 1350 and 1850, and preferentially between nucleotides 1350 and 1470 or 1650 and 1850, and which can amplify at least a part of the said gene, by cooperating with other primers having similar features and having a reversed polarity.

The said primers comprise prefentially from 10 to 40 and more preferentially from 10 to 30 nucleotides and their sequences can be one of the following ones :

SEQ ID1 (PL3) AAG TTA CCT TTG CTG CAT AAT CCC

SEQ ID2 (PL7) ATA GAT ACT CCA TAT CAT CCT GCT

SEQ ID3 (Plc) TCA AAA GAA TAT GCA AGA GGT

SEQ ID4 (PL1) TTCTAT CTT GGA GAGG CTATGCAC

SEQ ID5 (PL4) GCTACTAGTTCTTTTACATTCTTTCC.

The invention relates to combination of primers for the amplification of the gene encoding enterotoxin of C. perfringens, corresponding to a part of the sequence of the gene comprised between the nucleotides 450 and 550, and the primer SEQ ID7 (P146) GCT GGC TAA GAT TCT ATA TTT TTG TCC AGT which can amplify at least a part of the said gene.

The said primer corresponding to a part of the sequence between 450 and 550 comprised prefentially from 10 to 40 and more preferentially from 10 to 30 nucleotides and its sequence may be

SEQ ID6 (P145) GAA AGA TCT GTA TCT ACA ACT GCT GGT CC

The present invention relates moreover to a process for the determination of the presence of the gene encoding the enterotoxin, in a sample, comprising the following steps:
- the DNA from the sample is isolated ,
- parts of the genes encoding the lecithinase, or the enterotoxin, are amplified by polymerization chain reaction (PCR) with the help of specific primers, such as defined hereabove respectively for the lecithinase and for the enterotoxin,
- the amplification products are determined with the help of known methods.

Another object of the present invention is a process for the determination of the presence, and the numeration of C. perfringens in a sample, wherein:
- the DNA from the said sample is isolated ,
- parts of the genes encoding the lecithinase and the enterotoxin are amplified by polymerase chain reaction with the help of specific primers such as defined hereabove for respectively the lecithinase and the enterotoxin ,
- the amplification products are determined with the help of known methods.

The amplification products obtained by the process described hereabove can in particular be determined by electrophoresis on an agarose gel, followed by a transfer of the eluted DNA on "Nylon" membranes and by an hybridisation with a labelled probe specific to the amplified part of the gene. The labelled probe for the enterotoxin gene may be

SEQ ID8(EntA) GAA CGC CAA TCA TAT AAA TTT CCA GCT GGG

Other methods known by the man skilled in the art can also be used , if such methods are sufficiently specific and sensitive.

Such methods are in particular described in "The molecular cloning : A Laboratory Manual; SANDERS et al., Cold Spring Harbor Editor", in which the man skilled in the art can refer for carrying out the processes according to the present invention.

The choice of the hereabove described primers did not obviously emerge from the genes sequences such as published. Indeed, the man skilled in the art well knows that the choice of primers for the amplification of a given DNA part is difficult and that one can be faced with numerous difficulties.

For example, primers can lack of specificity or be weakly thermodynamicly stable.

The man skilled in the art can also be faced with other technical difficulties from different types. Thus, the choice that has to be made by the man skilled in the art in the sequence of the gene is difficult because of the important number of thinkable combinations between the two primers corresponding respectively to sequences of the two strands, from the DNA molecule which has to be amplified.

The use of softwares help the man skilled in the art in his choice but does not constitue a method leading automatically and obviously to primers permitting the sought amplification.

The primers such as defined hereabove are able to amplify the said genes if they cooperate with another primer having similar features but of reversed polarity. Indeed, the other primer must be situated on the strand of reversed polarity , called non-sense strand, in such a way that the polymerization of the two strands, initiated from the two primers produce two types of single strand DNA fragments which hybridate and form a double strand DNA molecule, called amplification product, which will be determined by known methods. The primer cooperating in the amplification of the fragment of the said gene have some similar features, i.e. that it is situated in the region of the said gene but on the strand of reversed polarity.

Preferentially, the combinations of the primers can be the following : PL1 and PL4, PL3 and PL7, P145 and P146, P145 and Ent A.

The processes of the invention hereabove described allow the detection of C. perfringens in a lot of samples, such as feces or food products, such as convenients foods, without necessiting a pretreatment of the sample.

In a general way, heating of the samples at temperature lysing the bacteriae and separating by centrifugation the DNA from bacterial fragments is sufficient.

In the case of food samples, in particular meats, it can be necessary to perform a pretreatment of the sample in order to eliminate substances which can interfer with the polymerase chain reaction.

Thus, the DNA can be isolated from a food sample by a process comprising the following steps:
- the sample is incubated in a medium allowing the growth of C. perfringens,
- the bacteriae are separated from the food particles by centrifugation,
- the bacteriae are put in contact with a resin which lyses them ,
- the DNA is separated from bacterial fragments.

Further, the application discloses a new C. perfringens beta toxine gene which has been identified, isolated and characterized.

Said gene, called β₂ gene, can be isolated from the C. Perfringens strains of C-type. Two corresponding plasmids were filed on December 12, 1994 at the CNCM Collection of Institut Pasteur and were registered respectively under the reference I-1499 for TGI/pMRP 109 and under the reference I-1500 for TGI/pMRP 126.

1 AAA GAA ATG GAT GCG TAT AGG AAA GTG ATG GAG AAT TAT CTT AAT GCT 48
1 K E M D A Y R K V M E N Y L N A 16

49 TTA AAA AAC TAC GAT ATT AAT ACA GTT GTA AAC ATT TCA GAA GAT GAA 96
17 L K N Y D I N T V V N I S E D E 32

97 AGA GTA AAT AAT GTT GAA CAG TAT AGA GAA ATG TTA GAA GAT TTT AAA 144
33 R V N N V E Q Y R E M L E D F K 48

145 TAT GAT CCT AAC CAA CAA CTG AAA TCT TTT GAA ATA CTT AAT TCA CAA 192
49 Y D P N Q Q L K S F E I L N S Q 64

193 AAG AGC GAT AAT AAA GAA ATA TTT AAT GTA AAA ACT GAA TTT TTA AAT 240
65 K S D N K E I F N V K T E F L N 80

241 GGT GCA ATT TAT GAT ATG GAA TTT ACT GTA TCA TCT AAA GAT GGA AAA 288
81 G A I Y D M E F T V S S K D G K 96

289 TTA ATA GTA TCT GAT ATG GAA AGA ACA AAA GTT GAG AAT GAA GGA AAA 336
97 L I V S D M E R T K V E N E G K 112

337 TAT ATT TTA ACA CCA TCA TTT AGA ACT CAA GTT TGT ACA TGG GAT GAT 384
113 Y I L T P S F R T Q V C T W D D 128

385 GAA CTA GCA CAA GCA ATT GGG GGA GTT TAT CCA CAA ACA TAT TCT GAT 432
129 E L A Q A I G G V Y P Q T Y S D 144

433 AGA TTT ACA TAT TAT GCA GAT AAT ATA TTA TTA AAC TTC AGA CAA TAT 480
145 R F T Y Y A D N I L L N F R Q Y 160

481 GCA ACT TCA GGT TCA AGA GAT TTA AAA GTA GAA TAT AGT GTT GTA GAT 528
161 A T S G S R D L K V E Y S V V D 176

529 CAT TGG ATG TGG AAA GAT GAT GTT AAA GCT TCT CAA ATG GTA TAT GGT 576
177 H W M W K D D V K A S Q M V Y G 192

577 CAA AAT CCT GAT TCT GCT AGA CAA ATA AGA TTA TAT ATA GAA AAA GGA 624
193 Q N P D S A R Q I R L Y I E K G 208

625 CAA TCT TTC TAT AAA TAT AGA ATA AGA ATT AAA AAC TTT ACA CCT GCA 672
209 Q S F Y K Y R I R I K N F T P A 224

673 TCA AT
225 S

pMRP109 is a recombinant plasmid containing the beginning of the beta toxin 2 gene.

pMRP109 was produced by insertion into the pUC19 vector restricted by SmaI of a DNA fragment amplified by PCR from the total DNA of strain CWC 245 of C. perfringens type C and primers deduced from the N-terminal protein sequence of beta 2 (P279) and an internal protein sequence (P280) :

P279 5'-AAA GAA ATI GAT GCI TAT AGI AAA GTI ATG G-5'
P280 5'-AT IGA IGC IGG IGT AAA ATT-3'.

The complete DNA inserted into pMRP109 was sequenced. This DNA corresponds to the beginning of the beta toxin 2 gene and codes for the first 225 amino acids of the functional protein.

pMRP126
GAT 528
D 176

529 CAT TGG ATG TGG AAA GAT GAT GTT AAA GCT TCT CAA ATG GTA TAT GGT 576
177 H W M W K D D V K A S Q M V Y G 192

577 CAA AAT CCT GAT TCT GCT AGA CAA ATA AGA TTA TAT ATA GAA AAA GGA 624
193 Q N P D S A R Q I R L Y I E K G 208

625 CAA TCT TTC TAT AAA TAT AGA ATA AGA ATT AAA AAC TTT ACA CCT GCA 672
209 Q S F Y K Y R I R I K N F T P A 224

673 TCA ATT AGA GTA TTT GGT GAA GGG TAT TGT GCA TAG AAA AAA ATA TGA 720
225 S I R V F G E G Y C A * 236

721 AGT GAC TTA GTC ACT TCA TAT TTT TTT TAC TAT TAA TTT TAT TAT ATA 768

769 AAA ACC TAA CAT ACA TGA AAG TAT TCT TAA TAC AGT TAT ATC AAA ATT 816

817 AAA GTA GGG GAA ATA AAA TAA AAG GCT AAA AAC TAT ATT AAA AAC TAT 864

865 AAA AAT TAT TAA ATT AGG 882

PMRP126 is a recombinant plasmid containing the end of the beta 2 gene.

pMRP126 was produced by cloning a DNA fragment of about 400 base pairs cleaved by Sau3A from C. perfringens type C strain CWC245 in the pUC19 vector cleaved by BamHI.

This part of the beta 2 gene codes for amino acids 176 to 236. Nucleotides 526 to 677 are common with the last 151 nucleotides of pMRP109.

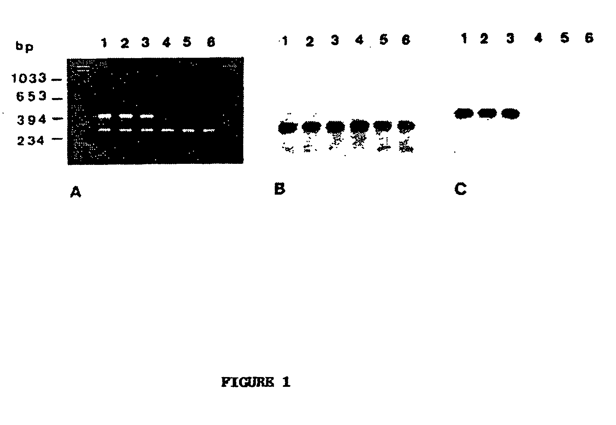

The present invention is illustrated without being limited by the following examples whereby Example 7 falls outside the scope of the invention in which figure 1 is an ethidium bromide stained agarose gel of amplification products obtained with enterotoxigenic C. perfringens type A strain 8-6 (1), type D strain IP76 (2), type E strain NCIB10748 (3), and non enterotoxigenic C. perfringens type A strain ATCC13124 (4), type B strain CN3922 (5) and type C strain CWC236 (6) (Panel A), and Southern hybridization with Plc (Panel B), and EntA (Panel C) probes.

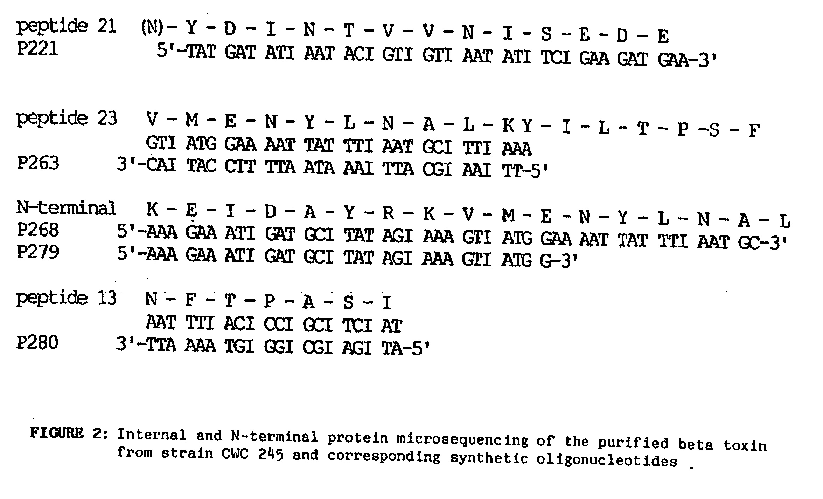

Figures 2-4 show sequences of the β2-toxin gene and protein.

### MATERIALS AND METHODS

### Bacterial strains

All bacterial strains used in this study are listed in table 1. The others C. perfringens strains were isolated from food poisoning outbreaks and feces of children suffering from diarrhoea ( see table 1).

Clostridium were grown, under anaerobic conditions, in TYG medium: trypticase (30 g/l), yeast extract (20 g/l), glucose (5 g/l) and cysteine HCl (0.5 g/l), pH 7,2. Strains were confirmed as C. perfringens by lactose fermentation, nitrate reduction, gelatinase production and absence of motility.

### Standard bacteriological methods

For bacteriological food and feces analysis, 10g of the samplee were weighted aseptically into sterile stomacher bags and homogenized for 2 minutes with 90 ml of peptoned water. One ml of decimal dilutions of the suspension was mixed with 9 ml SPS agar:. Tryptone ( 15 g/l), yeast extract (10 g/l), feric citrate (0.5 g/l), sodium sulfite (0.5 g/l), sodium thioglycolate (0.1 g/l), tween 80 (0.005 g/l), polymyxine B sulfate (0.001 g/l), disodic sulfadiazine (0.12 g/l) and agar (14 g/l pH 7). After a 18 h anaerobic incubation at 46°C the number of sulfito-reductor Clostridium was determined. Colonies surrounded with the.black characteristic precipitate were identified by biochemical test (lactose fermentation, nitrate reduction, gelatinase production and motility).

### Anti C. perfringens enterotoxin immunoglobulins

C. perfringens enterotoxin was purified from C. perfringens strain 8-6 and rabbit anti C. perfringens enterotoxin antibodies were prepared as previously described (POPOFF, 1984, FEMS MICROBIOL Let.21: 1-5). Anti C. perfringens enterotoxin immunoglobulins were purified by immunoaffinity. C. perfringens enterotoxin (3- 5 mg) was coupled to 1 g of cyanogen bromide - activated Sepharose 4B (Pharmacia, Paris, France) according to the instructions of the manufacturer. Five ml of rabbit anti C. perfringens enterotoxin serum were passed over the immuno affinity column. The column was washed with phosphate-buffered saline (PBS) until no protein was detected, and eluted with 1 M acetic acid. Fractions of 200 µl were collected into tubes containing 100 µl TRIS 3 M pH 8. The fractions containing anti C. perfringens enterotoxin immunoglobulins were dialyzed against PBS.

### Preparation of latex beads

Coating of latex beads (Bacto Latex 0.81, Difco, USA) was performed as described (SHAHRABADI et al. 1984, Clin. Microbiol. 20: 339-341). Latex suspension (2 - 5 ml) was diluted in 15 ml of glycine buffer (0.1 M glycine, 0.15 M NaCl, pH 8.2) containing 200 µg of specific anti C. perfringens enterotoxin immunoglobulins, and homogenized for 1 minute at room temperature. Then, 1 volume of PBS - 0.1 % bovine serum albumine (BSA) was added. The mixture was shaken by vortex mixing, and stored at 4°C. The negative latex control was prepared as the same, but using non immune IgG (Sigma, Paris, France).

### Slide latex agglutination test (SLAT) for C. perfringens enterotoxin detection.

SLAT was performed on a glass slide by mixing 25 µl of coated latex and 25 µl of serial ten fold dilutions of samples in PBS - 0,5 % BSA.

The mixture was gently rotated and the results were recorded after 3 minutes.

The enterotoxin detection in C. perfringens strains was carried out using SLAT and culture supernatant from cultures in the Duncan and Strong sporulation medium (DAMME-JONGSTEN et al. J. Clin. Microbiol. 28: 131-133).

Fecal specimens were diluted at 1 : 10 in PBS-0.5 % BSA, homogenized by vortexing, and centrifuged at 15 000 x g for 30 minutes. The clarified supernatant was tested by SLAT.

### Primers used in duplex PCR

The oligonucleotides for PCR amplification and for hybridization used in this study, the primers PL3, PL7, PL1, PL4 and Plc were derived from the C. perfringens alpha-toxin gene (11), the primers P145 and P146 and the probe Ent A were from the enterotoxin gene (VAN DAMME et al. 1989, précédemment cités).

All the oligonucleotides were chemically synthesized by using a nucleic acid synthesizer (model 380 B; Applied Biosystems Inc. Foster City, Calif.).

### Duplex PCR with broth culture

One ml of overnight bacterial cultures were transferred to a microcentrifuge tube and centrifuged at 12.000 x g for 3 minutes. The pellet was resuspended in 100 µl water, heated at 100°C for 10 minutes, and centrifugated at 12000 x g for 10 minutes. Five µl of the supernatant were submitted to PCR using a PTC-100 programmable Thermal Controller (M.J. Research Inc. Watertown, MA, USA). Each reaction tube contained 50 µl of a solution of 10 mM Tris-HCl pH 8.3, 50 mM KCl, 1.5 mM MgCl₂, 0.1 mg/ml gelatine, 200 µM of each of the deoxynucleotide (Boehringer Mannheim, Germany), 0.5µM of each primer and DNA sample. Evaporation within the tube was prevented by the addition of 100 µl mineral oil (Sigma, St-Louis, MO, USA). The reaction mixtures were treated to 94°C for 5 minutes to denature the DNA and then 2.5 units of Taq polymerase ( Boehringer) were added to each tube. All PCR reaction were performed using 45 cycles of the optimized thermal profile: 30 s denaturation at 94°C , 30 s primer-annealing at 55°C and 30 s primer-extension at 72°C. After the 45th cycle, the extension reaction was continued for another 10 minutes at 72°C, to ensure that the final extension steps was complete. Negative controls containing all reagents except template DNA were performed in each amplification set. To avoid contamination, sample preparation, PCR amplification and electrophoresis were performed in three different rooms.

### Duplex PCR with stool samples

Stool samples (0,1 g) were weighed aseptically into sterile tubes and homogenized with 0,9 ml of water. Samples were centrifugated at 12.000 x g for 3 minutes. The pellet was resuspended in 100 µl water, heated at 100°C for 10 minutes, centrifugated at 12000 x g for 10 minutes, and 5 µl of the supernatant were PCR amplified as described above.

### Pretreatment of food samples before the amplification reaction.

### a) Incubation of the sample.

The detection of viable bacteriae is of great importance in the food processes.

The discrimination between dead and alived bacteriae can be performed by cultivating the sample.

For the detection of C. perfringens, 25g of the sample are incubated in 225 ml of buffered peptonized water for 18 hours at 3.7°C. in anaerobiosis and without shaking.

The spores of C. perfringens are selected with the help of a thermal shock at 80°C of the food culture before incubating.

### b) Treatment of the sample for the PCR analysis.

After incubation of the food cultures, the food fragments are sedimentated and 1 ml of the enrichment medium is taken at the surface.

A low speed centrifugation (600 rpm) allows the elimination of the lowest food particles. The supernatent is recovered and centrifugated at a high speed ( 12 000 rpm) for 2 minutes. The supernatant is eliminated and the bacterial pellet is suspended in 2 ml of sterile water. The tube is then centrifuged at 12 000 rpm and the supernatant is eliminated with the help of a Pasteur pipette .

200 µl of the "InstaGene" matrix (commercialized by Biorad) are added on the pellet and incubated at 56°C for 30 minutes. The tube is vortexed during 10 seconds and put in a water bath at 100°C during 8 minutes. The tube is then vortexed for 10 seconds and centrifuged at 12 000 rpm for 3 minutes. 3 µl of the supernatant are taken in order to perform the duplex PCR which is finally performed on a volume of 50 µl.

Starting from 25 g of foods containing 10 or more than 10 C. perfringens allows the determination in the 15 µl of the amplified solution of the amplification product.

### Gel Electrophoresis

A 10 µl aliquot of amplified DNA was examined after electrophoresis through a composite agarose gel consisting of 3% NuSieve agarose and 1 % SeaKem agarose ( FMC BioProducts, Ropckland, (Me, USA) and containing 0.5 µg/ml ethidium bromide, in 1 x Tris-borate-EDTA buffer ( 0.089 M Tris, 0.089 M bioric acid, 0.002 M EDTA, pH 8). Electrophoresis was carried out for 1 hour a t 80 V. The gel was examined visually with an ultra-violet transilluminator.

### Hybridization procedure

DNAs electrophoresed on agarose gel were transferred to "Nylon" membranes (Boehringer, Paris, France) using 0.4 N NaOH and fixed by heating the membranes for 15 minutes at 120°C. Filters were pre-hybridized in 5 x Saline Sodium Citrate buffer (SSC), 0.1 % N-laurylsarcosine, 0.02 % Sodium Dodecyl Sulfate (SDS) in 1 % blocking reagent (Boehringer) at 65°C for 1 hour. Hybridization occurred with Ent A or Plc oligonucleotide 3'-endlabelled by digoxigenin; 11-DUTP with terminal deoxynucleotidyl-transferase (Boehringer). The probe was added to 50 ng/ml in the pre-hybridization solution and filters were incubated at 40°C for 1 hour. After 2 x SSC, 0.1% SDS washing, membranes were washed to a final stringency of 50°C in 0.1 x SSC, 0.1 % SDS and detection was performed with alkaline phosphatase-labelled anti-digoxigenin antibody (Boehringer), nitroblue tetrayolium (NBT) and bromo-4-chloro-3-indolyl phosphate (BCIP) according to the instruction of the manufacturer (Boehringer).

### EXAMPLE 1:

### C. perfringens duplex PCR specificity and characterization of the enterotoxigenic strains.

The specificity of the duplex PCR with the two primers sets derived from the phospholipase C and CPE genes was assessed by using different Clostridium species and various bacterial species frequently encountered in foods (table 1). The 283 bp amplification product was observed in ethidium bromide stained agarose gel with all C. perfringens strains tested (table 1). This amplification product had the expected size of the phospholipase C gene DNA fragment generated by PL3 and PL7 primers set. This result was confirmed by DNA-DNA hybridation with the internal primer Plc (figure 1).

The other bacterial strains tested showed no amplification products with the two primers sets PL3-PL7 and P145-P146 (table 1).

Seven C. perfringens strains out of 24 showed an additional 426 bp amplification product which corresponded to the expected size of the CPE DNA fragment delimited with P145 and P146. This 426 bp amplification product hybridized with the Ent A primer localized between P145 and P146 on the CPE DNA sequence and not with Plc primer derived from the phospholipase C gene ( figure 1).

The C. perfringens strain 8.6 was found enterotoxinogenic by the duplex PCR amplification (table 1). This data is in agreement with the fact that this strain produced CPE using Vero cell cytotoxicity , mouse lethality and immunoprecipitation.

### EXAMPLE 2:

### Sensitivity of the duplex PCR using broth cultures.

The duplex PCR was performed directly with broth cultures of the enterotoxogenic C. perfringens strain 8.6, in order to determine the sensitivity of the method. The number of bacteriae in culture samples was determined microscopically using a Petrov Chamber. The PCR amplification was carried out as described in material and methods. It was found that as few as 50 bacteria in the reaction volume yielded positive results after agarose gel electrophoresis and ethidium bromide staining. The sensitivity was 10 fold increased when the amplification products electrophoresed on agarose gel were transferred to nylon membrane and hybridized with digoxigenin labelled internal probes Ent A and Plc. In these condituions, the detection limit was approximately 5 bacteria in the reaction volume of the duplex PCR.

### EXAMPLE 3

### Application of the duplex PCR to stool samples.

Twenty three stool samples originated from a suspected C. perfringens food borne intoxication outbreak were investigated directly by the duplex PCR and standard microbiological methods for C. perfringens detection. Eighteen samples were found positive in the duplex PCR (table 2). All these samples yielded two amplifications products which has the expected size of the DNA fragments delimited by PL3-PL7 and P145 - P146, and hybridized with the internal primers Plc and Ent A respectively, signifying that they contained enterotoxigenic C. perfringens. The sulfito reductor bacteria counted by the standard bacteriological methods ranged from less than 10⁴ to 10⁸ bacteria per gram. Five feces samples were found negative in the duplex PCR and DNA-DNA hybridization test. The corresponding sulfito-reductor bacteria counting was less than 10⁴ per gram for 3 samples, and 10⁵ to 2.10⁵ for the two other (table 2).

### EXAMPLE 4

### C. perfringens enterotoxin SLAT in stool samples.

The CPE was detected in 18 stool samples out of 23 by SLAT as described in material and methods (table 2). Enterotoxigenic C. perfringens was identified by the duplex PCR in 17 of these 18 SLAT positive stool samples. Four samples were negative with both tests.

One stool sample was shown to contain enterotoxigenic C. perfringens by the duplex PCR and no CPE by SLAT. The low number of enterotoxigenic C. perfringens (less than 10⁴ per gram) explained the absence of CPE in this example.

CPE was detected by SLAT in one stool sample (table 2) which was negative in the duplex PCR. The level of sulfito reductor bacteria in this sample was 10⁵ per gram. However, twenty C. perfringens clones isolated on sheep blood agar, were identified as enterotoxigenic C. perfringens by the duplex PCR.

### EXAMPLE 5:

### Application of the duplex PCR to naturally and artificially contaminated food samples.

The food sample which was responsible of the food intoxication outbreak was found to contain enterotoxigenic C. perfringens by the duplex PCR performed directly without enrichment culture. The counting of sulfitoreductor bacteria was 10⁵ per g, and no CPE was detected by SLAT.

In order to investigate the sensitivity of this method, the duplex PCR was assayed directly with artificially contaminated food samples. The detection limit was found to be 10⁵ C. perfringens per g. The sensitivity was improved by using an overnight enrichment culture as described in material and methods. Among 59 naturally contaminated food samples, 2 were found to contain 5.10⁵ and 10³ C. perfringens per g respectively by the classical method and were positive by the duplex PCR.

All the 57 food samples which did not contain sulfitoreductor bacteria and were artificially contaminated (10 C. perfringens per g) gave a positive result in duplex PCR after enrichment culture (table 3).

### CONCLUSION

All the 24 C. perfringens strains tested yielded the 426 bp DNA fragment hybridizing with the probe Plc, which corresponds to the presence of the phospholipase C gene. Seven of these C. perfringens strains ( 2 type A reference strains, 3 type A strains isolated from food, and 2 type D strains) showed an additional amplification product hybridizing with Ent A probe, which indicates the presence of the CPE gene.

The duplex PCR is specific of C. perfringens. No amplification product was observed with 27 Clostridium strains different from C. perfringens and 20 other bacteria species frequently encounted in foods.

An important advantage of this method is to identify easily and quickly the enterotoxigenic C. perfringens strains.

The duplex PCR can be used with C. perfringens grown in regular culture medium for anaerobic bacteria without DNA purification. The sensitivity of the duplex PCR using pure culture is 50 bacteria by visualization of ethidium bromide stained amplification product in agarose gel, and 5 bacteria in the reaction volume by DNA-DNA hybridization with the internal probes.

This duplex PCR DNA-DNA hybridization assay was tested with biological and food samples. Enterotoxigenic C. perfringens were detected directly in 18 among 23 stools samples from patients suffering food borne intoxication. These results were in agreement with the numeration of the sulfito reductor bacteria by the standard method and the detection of the CPE by SLAT, except for two stools samples. One stool sample containing 10⁵ sulfito reductor bacteria per gram and CPE by SLAT was found negative by the duplex PCR. But, individual C. perfringens clones isolated from this stool sample were identified as enterotoxigenic C. perfringens by the duplex PCR. The presence of PCR inhibitors in this sample accounts probably for this negative result. The other stool sample showed a low number of sulfito-reductor bacteria and no detectable CPE, but was positive in the duplex PCR. A contamination of this sample could explain the positive result in duplex PCR.

The detection limit of enterotoxigenic C. perfringens in stools by the duplex PCR is 10⁴ and 10⁵ per gram. This sensitivity is suitable for the diagnosis of C. perfringens food borne intoxication, since it has been reported that C. perfringens is recovered at high number (10⁶ and more per g) in stools from patients suffering C. perfringens food borne intoxication.

Controls of food C. perfringens contamination constitute an important step in the prevention of food born intoxication by this microorganism. The standard method routinely used detects sulfito-reductor bacteria which encompasse C. perfringens and also other Clostridium. The duplex PCR is specific of C. perfringens and discriminates the enterotoxigenic strains which are the causative agents of C. perfringens food borne intoxication. The sensitivity of this method (10 C. perfringens per g) performed with enrichment cultures of food samples is compatible with the prescribed detection levels for food controls. The limit of the duplex PCR directly with food samples without enrichment cultures is 10⁵ bacteria per g , the duplex PCR can be used directly when a C. perfringens food borne intoxication is suspected.

The duplex PCR is more rapid and simple than the standard procedure which needs additional bacteriological tests for the identification of the suspected clones. This method can be applied to food controls for the detection of C. perfringens and identification of the enterotoxigenic strains.

### EXAMPLE 6:

Further experiments illustrating the application of the duplex PCR according to the invention to the detection of C. perfringens in foodstuffs, are presented hereinbelow.

### 1) ANIMAL FEED

Several samples of animal feed have been analyzed for the presence of C. perfringens. These feeds were made up of meat, fish or bone meals. The concentration of sulfito-reductor anaerobic organisms cultivated at 46°C have been determined in 44 naturally contaminated foods and more precisely the presence of C. perfringens spores has been ascertained by use of LS medium (BEERENS H, Romond Ch., Lepage C., Criquelion J. (1982). A liquid medium for the enumeration Clostridium perfringens in food and faeces. In "Isolation and identification methods for foods poisons organisms", pp. 137-149. Ed. Corry J.E.L., Roberts D. and Skinner F.A. Academic Press London) and by the PCT duplex technique described previously. Spore selection was carried out by thermal shock treatment at 80°C for 10 minutes. The presence of inhibitors for the gene amplification reaction (PCR) led to use an extraction and purification procedure for the nucleic acids which involved the "Insta Gen" (Bio-Rad) matrix and which has been described previously.

On the 44 samples analyzed (table 4) 12 were positive according to the procedure using the LS medium and the PCR duplex test. 24 samples were negative with both methods. 36 samples out of the 44 analyzed gave the same results for the two methods, giving a correlation rate of over 80% between the bacteriological method and the genetic test. For 5 samples the PCR duplex method seemed to be more sensitive than the procedure using the LS medium. For these 5 samples it was possible to isolate, from the thioglycolate liquor incubated for 48 hours, at least one colony on TSC medium which had the biochemical characteristics of C. perfringens. The low concentration of C. perfringens (< 10/g) in the samples was only detected by the PCR technique, which was more sensitive than the procedure using thee LS medium.

Three samples were found to be positive with the procedure using LS medium but stayed negative with the PCR duplex test. These samples contained respectively 7.2 and 1 ASR per gram. In order to ascertain whether the PCR duplex test had not been compromised by the presence of inhibitors remaining after the nucleic acid extraction phase, a few nanograms of C. perfringens DNA (2 ng) were added to the extracts obtained from the 3 samples and the gene amplification test was again carried out under the same conditions. The 3 tests then became positive showing that no inhibitor effect had been observed initially. In addition, the 10 ASR colonies corresponding to these 3 samples were subjected to biochemical identification and a PCR duplex test. These 2 analyses still remained negative, showing evidence of the absence of C. perfringens in these samples. In fact, bearing in mind the difficulties in interpretation of the method described by BEERENS and al. (the presence of gas in 1/3 of the Durham belljar and the presence of a black precipitate at the bottom of the tube were sometimes evaluated differently according to the users) and the very doubtful results obtained for these 3 samples, it seems that these 3 samples correspond to false positives in the method described by BEERENS and al.

### 2) HUMAN FOODSTUFFS

Within the framework of systematic monitoring of food hygiene, the number of C. perfringens which can be identified is generally low (< 10² per gram). Various foods (cooked dishes, beef , cooked meats, fish, powdered milk) were analyzed using the PCR duplex technique and the results were compared with those obtained by the standard bacteriological method (standard method ISO 7937).

Only 3 samples were found to be positive according to the two methods, the other 27 containing no C. perfringens. The PCR test allowed the detection of C. perfringens in 24 hours, while the bacteriological method was much longer (48-72 h). The foods concerned were packs of a frozen ground beef and potato dish. They were contaminated by type A non-enterotoxinogenic C. perfringens.

### EXAMPLE 7:

### STUDY OF THE C. PERFRINGENS BETA TOXIN GENE

In order to apply a PCR type method to the identification of type C C. perfringens strains responsible for necrotic enteritis, it was necessary to determine the sequence of the beta toxin gene.

The applicant carried out the production and purification of the beta toxin from the CWC 245 strain of C. perfringens, then determined peptide sequences, produced oligonucleotide markers and finally carried out the cloning and sequencing of this gene.

### (1) - Production and purification of the beta toxin.

Cultures were made on TGY (Trypticase 30 g/l, yeast extract 20 g/l, glucose 25 g/l, pH 7.5) in a 2 l fermenter with continuous nitrogen flow, moderate stirring and regulation of pH at 7.5.

After 5 hours at 37°C, cholesterol in alcoholic solution (20 mg/l) was added to the culture in order to precipitate the theta toxin. The culture was centrifuged at 10 000 g at 4°C for 20 minutes. The supernatant was precipitated by 60% saturated ammonium sulfate. After centrifuging at 20 000 for 20 minutes, the residue was dissolved in PBS (pH 6.8) and reduced with 50 mM of dithiothreitol. The sample was added in 3 fractions to a biogel P4 (Bio-rad) column equilibrated in PBS. The eluted proteins were applied to a column of thiopropyl-Sysharon 6B (58g of resin expanded in PBS) and a flow of 5 ml/h. The column was washed with 100 ml of PBS. Under these conditions the beta and delta toxins were not retained on the column, while the theta and alpha toxins were retained. The effluent was concentrated by ultrafiltration on a PM 10 (Millipore) membrane and submitted to preparative iso-electrofocalization (16 000 V for 16 hours) with a pH gradient of 3.5 to 8. The material focalized between pH 5.3 and 5.5 was collected and concentrated by ultrafiltration.

The concentrated fraction was added to a column of Biogel P100 (Biorad) equilibrated in PBS and the different fractions were tested by electrophoresis in polyacrylamide-SDS gel. In this way the beta toxin was purified to homogeneity (MW 28 000 and pH 5.4 - 5.5).

The purified beta toxin was lethal to mice (4 µg, IV), dermonecrotic after injection into guinea-pig skin (2 µg), and enteronecrotic after injection into rabbit intestinal loop (40 µg). The enterotoxic effect was inhibited by incubation of the beta toxin with trypsin.

### (2) Protein microsequencing of the beta toxin and oligonucleotides.

The purified beta toxin was electrophoresed on polyacrylamide-SDS, then transferred onto Immobilon membrane and subjected to microsequencing of the N-terminal region.

The internal protein sequences were obtained by tryptic digestion of the beta toxin, purification of the different peptides by HPLC chromatography and microsequencing of peptides 21, 23 and 13.

Oligonucleotides deduced from the protein sequences were synthesized taking into account of the normal codon of Clostridium and with inosines (I) in the most degenerate positions (figure 2).

### (3) Hybridization of the oligonucleotides.

The synthetized oligonucleotides were tested for hybridization with total DNA of CWC 245 digested by different restriction enzymes. The hybridizations were carried out over 16 hours at 40°C with oligonucleotides labeled with ²²P by polynucleotide kinase and washings were carried out in 6 X SSC at 40°C.

After autoradiography, these oligonucleotides did not clearly reveal a specific DNA fragment. However, clonings were carried out and the' clones, identified with the help of the oligonucleotides , did not contain the DNA fragments sought for after verification by nucleotide sequencing.

### (4) Amplification of a DNA fragment of the beta toxin gene by PCR.

Since the production of recombinant clones containing the beta toxin gene was not possible by conventional cloning methods, this gene was amplified by PCR. To do this, an oligonucleotide (P 279) deduced from the N-terminal protein sequence of the beta toxin and an oligonucleotide (P 280) deduced from an internal protein sequence (peptide 13) but in the reverse direction were used.

A DNA fragment of about 600 bp was thus amplified - specifically from the total DNA of CWC 245.

The 600 bp DNA fragment was cloned in the pUC19 vector (pMRP 109 recombinant plasmid) and then sequenced. It contained an open reading framework and the terminal portions corresponded to the protein sequences obtained by protein microsequencing (N-terminal and peptide 13), showing that the cloned DNA fragment corresponded well to the gene sought for.

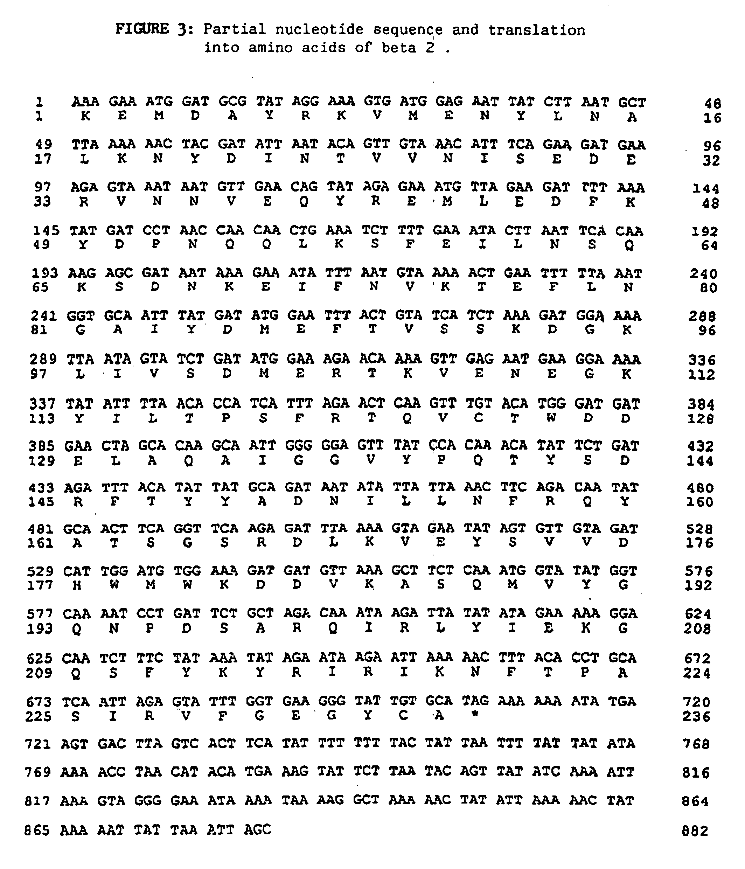

The 3' part of this gene was cloned in pUC 19 using the total DNA of CWC 245 digested by Sau 3A and identified with the P 311 marker whose sequence is localized in the 3' part of insert of pMRP 109. The pMRP 126 recombinant clone was sequenced and contained the stop codon of the beta toxin gene and a consensus transcription termination sequence. The sequence of the beta toxin gene from strain CWC 245 and its translation into amino acids are given in figure 3.

### (5) Comparison of the beta toxin gene from strain CWC 245 with that from strain NCTC 8533 and genetic analysis of the type C strains.

Titball et al (FEMS Microbiology Letters 97 (1992) 77-82) recently published the cloning and sequencing of the beta toxin gene from C. perfringens type B strain NCTC 8533.

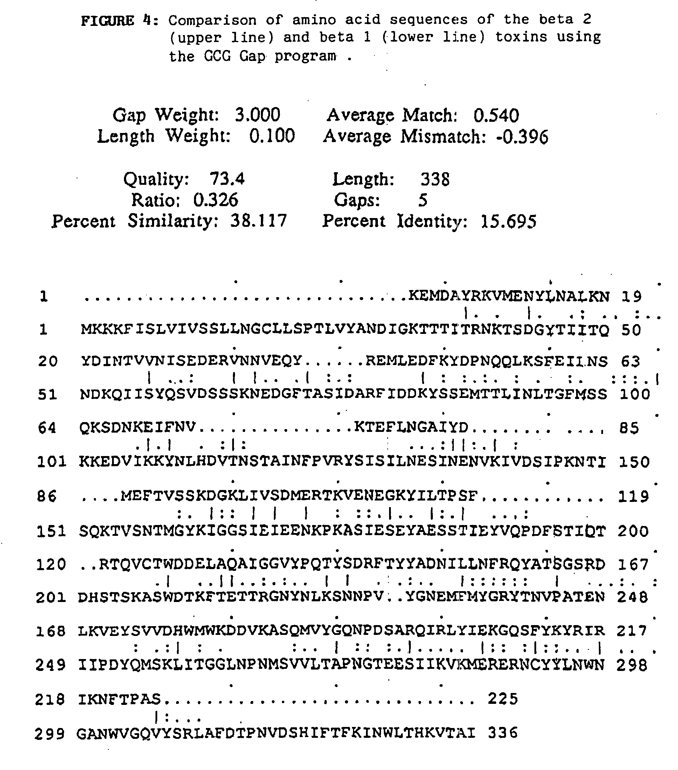

Comparison of the protein sequence of the beta toxin from strain NCTC 8533 (provisionally referred to as beta 1) with that of the beta toxin gene from strain CWC 245 (provisionally referred to as beta 2) showed no significant homology (figure 4).

The role of beta toxin 2 as a virulence factor has not yet been clearly established.

The presence of the beta 1 and beta 2 genes was searched for by PCR in a series of type B and C C. perfringens strains (Table 1).

PCR conditions:
- Buffer :
   - TRIS 10 mM pH 8.2
   - KCl 50 mM
   - dNTP 100 mM
   - Bovine serum albumin 0.1 mg/ml
   - Beta-mercaptoethanol 10 mM
   - Primers 50 pmoles of each per reaction
   - TAQ polymerase (Amersham) 0.5 µ
- Primers:
   - Primers for beta 2
   - P319 5'-GGAAAGTGATGGAGAATTATCTTAATGC-3'
   - P320 5'-GCAGAATCAGGATTTTGACCATATACC-3' (reverse)
      573 bp amplified fragment.
- Primers for beta 1
- BetatoxL 5'-AGGAGGTTTTTTTATTTTTTTTTTTGAAG-3'
- BetatoxR 5'-TCTAAATAGCTGTTACTTTGTG-3' 962 bp amplified fragment.
- Amplification cycles
- initial denaturation, 2 min at 94°C
- 30 cycles comprising -20 s at 94°C
- 20 s at 50°C
- 20 s at 72°C
- 5 minutes at 72°C
   PREM III thermocycler apparatus (Flobio)
- Amplification products analysis by electrophoresis on 1 % agar gel containing ethidium bromide and detection by UV transillumination.

These strains were identified by the standard method on mice using anti C. perfringens neutralizing serum (Welcome) according to the currently recognized nomenclature; all these strains produced the beta toxin.

Genetic analysis showed that the 3 B strains possessed the beta 1 gene and that the type C strains fell into 3 groups: those with the beta 1 gene, those with the beta 2 gene, and those with both (table 5).

The above experimental results show that the beta toxin gene from strain CMC245 differs from the beta toxin gene from strain NCTC 8533.

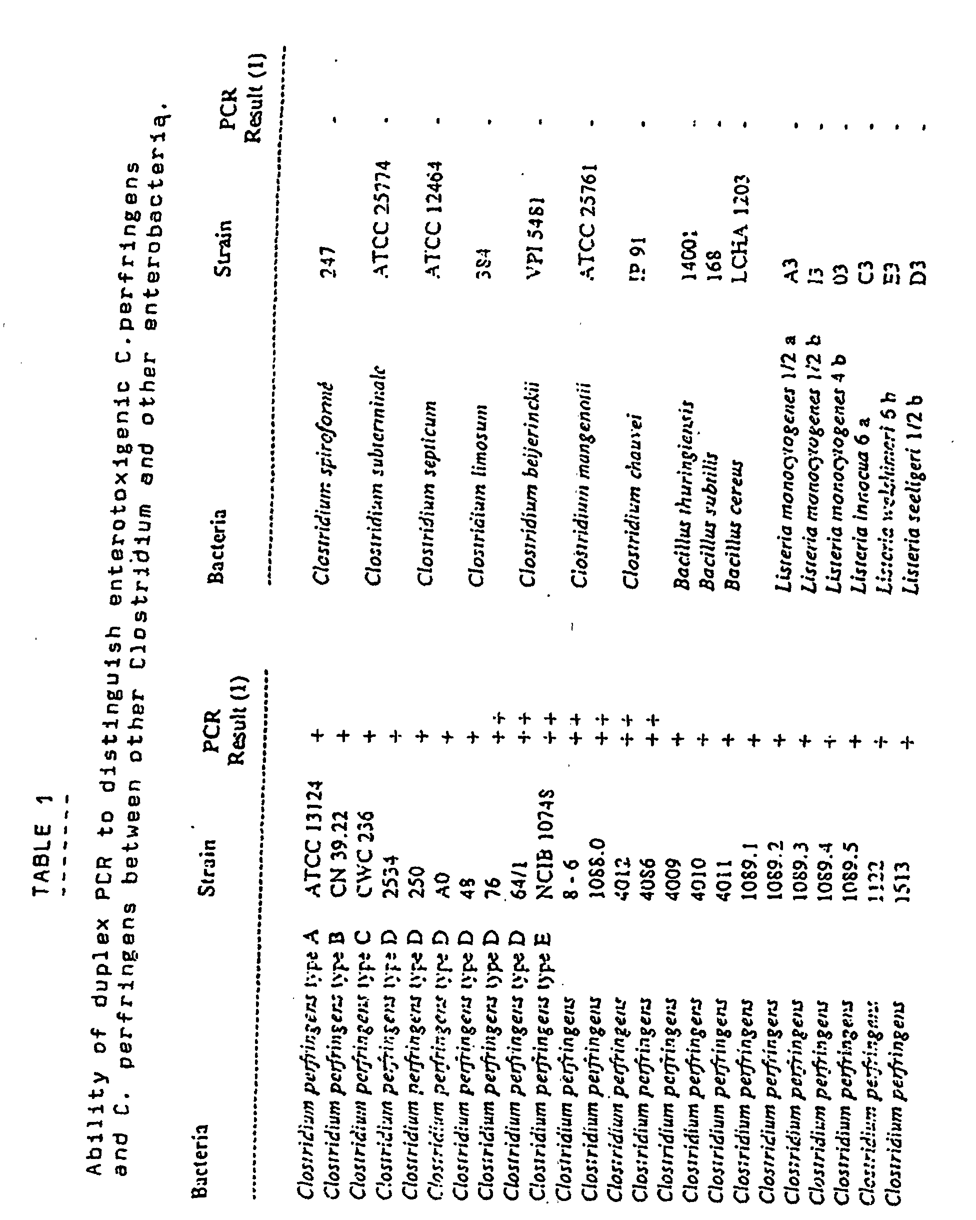

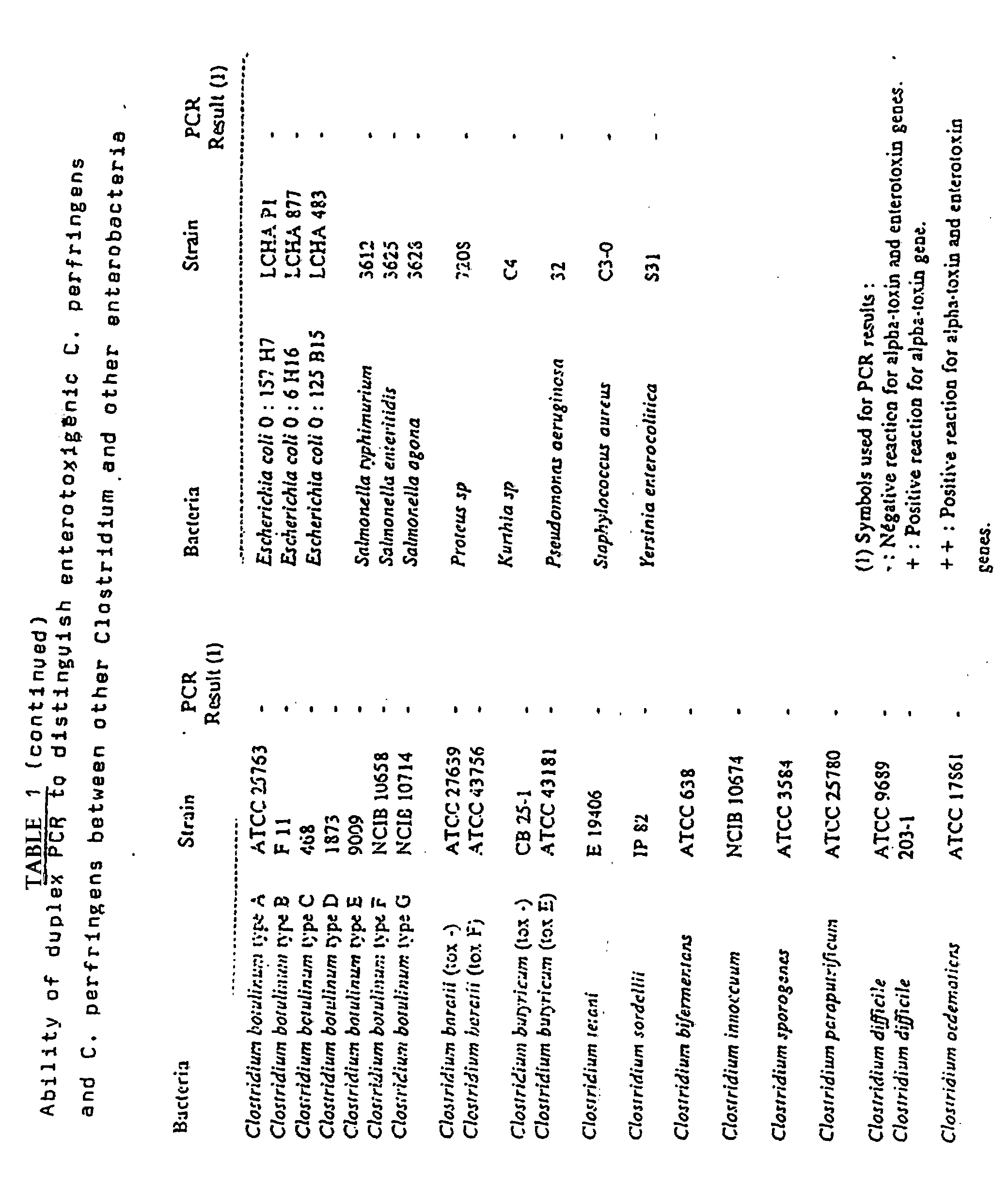

**Table 2:**

| Stool samples investigation | | | |
|---|---|---|---|
| Samples | Number of sulfito-reductor bacteria | Duplex PCR | SLAT |
| 1 | < 10⁴ | - | - |
| 2 | < 10⁴ | - | - |
| 3 | < 10⁴ | - | - |
| 4 | < 10⁴ | + | - |
| 5 | 10⁴ | + | + |
| 6 | 10⁴ | + | + |
| 7 | 10⁴ | + | + |
| S | 2.10⁴ | + | + |
| 9 | 5.10⁴ | + | + |
| 10 | 5.10⁴ | + | + |
| 11 | 10⁵ | - | + |
| 12 | 10⁵ | + | + |
| 13 | 10⁵ | + | + |
| 14 | 2.10⁵ | - | - |
| 15 | 2.10⁵ | + | + |
| 16 | 8.10⁵ | + | + |
| 17 | 10⁶ | + | + |
| 18 | 2.10⁶ | + | + |
| 19 | 2.10⁶ | + | + |
| 20 | 10⁷ | + | + |
| 21 | 10⁷ | + | + |
| 22 | 10⁷ | + | + |
| 23 | 10⁷ | + | + |

**Table 3.**

| C. perfringens investigation in food samples | | | | | |
|---|---|---|---|---|---|
| | C. perfringens investigation in naturally contaminated food samples | | | C. perfringens investigation in artificially contaminated food samples | |
| Food sample | Total | Classical method | Duplex PCR | Total | Duplex PCR |
| Cooked food | 31 | 1(5x10⁵) | 1 | 30 | 30 |
| Pork butchery | 13 | 1 (10³) | 1 | 12 | 12 |
| Raw meat | 11 | 0 | 0 | 11 | 11 |
| Milk product | 2 | 0 | 0 | 2 | 2 |
| Salad | 2 | 0 | 0 | 2 | 2 |

**TABLE 4:**

| Comparison between LS medium and the PCR technique in the detection of C. perfringens | | | |
|---|---|---|---|
| N = 44 animal foods (bone meal, meat or fish meal) | | | |

| | | LS MEDIUM | (Beerens) |
|---|---|---|---|
| | | + | - |
| PCR | + | 12 | 5 * |
| | - | 3** | 24 |

| | | | |
|---|---|---|---|
| * ASR concentration < 10/g At least 1 colony identified as C. perfringens after isolation on TSC medium. | | | |
| ** No colony isolated on TSC medium was identified as C. perfringens. | | | |

**TABLE 5**

| PCR identification of the genes for beta toxin 1 sequenced from strain NCTC 8533 and beta toxin 2 sequenced from strain CWC 245 in different strains of type B and C C. perfringens identified by the standard method | | |
|---|---|---|
| | Beta 1 | Beta 2 |
| C. perfringens B | | |
| NCTC8533 | + | - |
| NCTC6121 | + | - |
| IP278-220 | + | - |
| CN3922 | + | - |

| C. perfringens C | | |
|---|---|---|
| ATCC3628 | + | - |
| NCTC8O81 | + | - |
| NCTC3180 | + | + |
| NCTC3182 | + | + |
| NR866 | + | + |
| NR316 | + | + |
| CWC236 | + | + |
| CWC239 | + | + |
| CWC240 | + | + |
| CWC241 | + | + |
| CWC243 | + | + |
| IP232-87 | + | + |
| IP692-83 | + | + |
| CWC235 | - | + |
| CWC237 | - | + |
| CWC245 | - | + |
| CWC249 | - | + |
| S608 | - | + |
| IP93R | - | + |

## Claims

1. A combination of primers for the amplification of at least one part of the gene encoding the enterotoxin of C. perfringens, comprising:
- a primer which corresponds to a part of the sequence comprised between the nucleotides 450 and 550 of the enterotoxin gene;
- the primer

SEQ ID7 (P146) GCT GGC TAA GAT TCT ATA TTT TTG TCC AGT.

2. A combination of primers according to claim 1, **characterized in that** the primer which corresponds to a part of the sequence comprised between the nucleotides 450 and 550; of the enterotoxin gene comprises almost from 10 to 30 nucleotides.

3. The combination of primers according to claim 1, wherein the primer which corresponds to a part of the sequence comprised between the nucleotides 450 and 550 consists of the primer

SEQ ID6 (P145) GAA AGA TCT GTA TCT ACA ACT GCT GGT CC

4. A combination of primers according to any one of claims 1 to 3 that comprises additionally the primer

SEQ ID8 (EntA) GAA CGC CAA TCA TAT AAA TTT CCA GCT GGG

5. A process for the detection of the gene encoding the enterotoxin in a sample, comprising the following steps:
- DNA is isolated from the sample,
- the parts of the genes encoding the enterotoxin are amplified by chain polymerisation with the help of the primers, according to one of the claim 1 to 3.
- the amplification products are determined with the help of known methods.

6. A process for the determination of the presence and the numeration of C. perfringens in a sample, comprising the following steps:
- DNA from the sample is isolated,
- parts of the genes of C perfringens encoding respectively the lecithinase and the enterotoxin are simultaneously amplified by chain polymerization with the help of specific primers, corresponding respectively to:
• those defined in one of claims 1 to 3, for the amplification of the enterotoxin gene, and
• to a part of the sequence comprised between nucleotides 1350 and 1850 and preferentially between nucleotides 1350 and 1470 or between nucleotides 1650 and 1850 of the lecithinase gene, which is able to amplify a part of the said gene, by co-operating with another primer having similar features and a reversed polarity, for the amplification of at least a part of the lecithinase gene, and
• the amplification products are determined with the help of known methods.

7. The process according to claim 6 **characterised in that** the primers for the amplification of at least a part of the lecithinase gene comprises almost from 10 to 30 nucleotides.

8. The process according to one of the claims 6 and 7 **characterized in that** the primers for the amplification of at least a part of the lecithinase gene comprise one of the following sequences:

SEQ ID1(PL3) AAG TTA CCT TTG CTG CAT AAT CCC

SEQ ID2 (PL7) ATA GAT ACT CCA TAT CAT CCT GCT

SEQ ID3 (Plc) TCA AAA GAA TAT GCA AGA GGT

SEQ ID4 (PL1) TTC TAT CTT GGA GAGG CTATGCAC

SEQ ID5 (PL4) GCTACTAGTTCTTTTACATTCTTTCC

9. A process according to one of the claims 5 to 8 wherein the DNA is isolated from a food sample according to the following steps:
- the sample is incubated in a medium allowing the growth of C. perfringens,
- the bacteriae are separated from the food particles by centrifugation,
- the bacteriae are put in contact with a resin lysing them, and
- the DNA is separated from the bacterial fragments.

## Patentansprüche

1. Kombination von Primern für die Amplifikation wenigstens eines Teils des Gens, das das Enterotoxin von *C. perfringens* codiert, umfassend:
einen Primer, der einem Teil der Sequenz entspricht, die zwischen den Nucleotiden 450 und 550 des Enterotoxin-Gens enthalten ist;
den Primer SEQ ID Nr. 7 (P146) GCT GGC TAA GAT TCT ATA TTT TTG TCC AGT.

2. Kombination von Primern gemäß Anspruch 1, **dadurch gekennzeichnet, dass** der Primer, der einem Teil der Sequenz entspricht, die zwischen den Nucleotiden 450 und 550 des Enterotoxin-Gens enthalten ist, fast 10 bis 30 Nucleotide umfasst.

3. Kombination von Primern gemäß Anspruch 1, wobei der Primer, der einem Teil der Sequenz entspricht, die zwischen den Nucleotiden 450 und 550 des Enterotoxin-Gens enthalten ist, aus dem Primer SEQ ID Nr. 6 (P145) GAA AGA TCT GTA TCT ACA ACT GCT GGT CC besteht.

4. Kombination von Primern gemäß einem der Ansprüche 1 bis 3, die zusätzlich den Primer SEQ ID Nr. 8 (EntA) GAA CGC CAA TCA TAT AAA TTT CCA GCT GGG umfasst.

5. Verfahren zum Nachweis des Gens, das das Enterotoxin codiert, in einer Probe, umfassend die folgenden Schritte:
- DNA wird aus der Probe isoliert;
- die Teile der Gene, die das Enterotoxin codieren, werden durch Kettenpolymerisation mit Hilfe der Primer gemäß einem der Ansprüche 1 bis 3 amplifiziert;
- die Amplifikationsprodukte werden mit Hilfe bekannter Verfahren bestimmt.

6. Verfahren zur Bestimmung der Anwesenheit und der Anzahl von *C. perfringens* in einer Probe, umfassend die folgenden Schritte:
- DNA wird aus der Probe isoliert;
- Teile der Gene von *C. perfringens,* die die Lecithinase bzw. das Enterotoxin codieren, werden gleichzeitig durch Kettenpolymerisation mit Hilfe spezieller Primer, die jeweils den folgenden entsprechen, amplifiziert:
- den in einem der Ansprüche 1 bis 3 definierten zur Amplifikation des Enterotoxin-Gens; und
- einem Teil der Sequenz, die zwischen den Nucleotiden 1350 und 1850 und vorzugsweise zwischen den Nucleotiden 1350 und 1470 oder zwischen den Nucleotiden 1650 und 1850 des Lecithinase-Gens liegt und die in der Lage ist, einen Teil des Gens zu amplifizieren, indem sie mit einem anderen Primer kooperiert, der ähnliche Merkmale und eine umgekehrte Polarität aufweist, zur Amplifikation wenigstens eines Teils des Lecithinase-Gens; und
- die Amplifikationsprodukte werden mit Hilfe bekannter Verfahren bestimmt.

7. Verfahren gemäß Anspruch 6, **dadurch gekennzeichnet, dass** die Primer für die Amplifikation wenigstens eines Teils des Lecithinase-Gens fast 10 bis 30 Nucleotide umfassen.

8. Verfahren gemäß einem der Ansprüche 6 und 7, **dadurch gekennzeichnet, dass** die Primer für die Amplifikation wenigstens eines Teils des Lecithinase-Gens eine der folgenden Sequenzen umfassen:

SEQ ID1(PL3) AAG TTA CCT TTG CTG CAT AAT CCC

SEQ ID2 (PL7) ATA GAT ACT CCA TAT CAT CCT GCT

SEQ ID3 (Plc) TCA AAA GAA TAT GCA AGA GGT

SEQ ID4 (PL1) TTC TAT CTT GGA GAGG CTATGCAC

SEQ ID5 (PL4) GCTACTAGTTCTTTTACATTCTTTCC

9. Verfahren gemäß einem der Ansprüche 5 bis 8, wobei die DNA gemäß den folgenden Schritten aus einer Lebensmittelprobe isoliert wird:
- die Probe wird in einem Medium inkubiert, das das Wachstum von *C. perfringens* ermöglicht;
- die Bakterien werden durch Zentrifugation von den Lebensmittelteilchen abgetrennt;
- die Bakterien werden mit einem Harz in Kontakt gebracht, das sie lysiert; und
- die DNA wird von den Bakterienfragmenten abgetrennt.

## Revendications

1. Combinaison d'amorces pour l'amplification d'au moins une partie du gène codant pour l'entérotoxine de C. perfringens, comprenant:
- une amorce qui correspond à une partie de la séquence comprise entre les nucléotides 450 et 550 du gène de l'entérotoxine;
- l'amorce

SEQ ID7 (P146) GCT GGC TAA GAT TCT ATA TTT TTG TCC AGT.

2. Combinaison d'amorces selon la revendication 1, **caractérisée en ce que** l'amorce qui correspond à une partie de la séquence comprise entre les nucléotides 450 et 550 du gène de l'entérotoxine comprend presque de 10 à 30 nucléotides.

3. Combinaison d'amorces selon la revendication 1, dans laquelle l'amorce qui correspond à une partie de la séquence comprise entre les nucléotides 450 et 550 est constituée de l'amorce

SEQ ID6 (P145) GAA AGA TCT GTA TCT ACA ACT GCT GGT CC

4. Combinaison d'amorces selon l'une quelconque des revendications 1 à 3, qui comprend en outre l'amorce

SEQ ID8 (EntA) GAA CGC CAA TCA TAT AAA TTT CCA GCT GGG

5. Procédé pour la détection du gène codant pour l'entérotoxine dans un échantillon, comprenant les étapes suivantes:
- l'ADN est isolé à partir de l'échantillon,
- les parties des gènes codant pour l'entérotoxine sont amplifiées par polymérisation en chaîne à l'aide des amorces, selon l'une des revendications 1 à 3,
- les produits d'amplification sont déterminés à l'aide de procédés connus.

6. Procédé pour la détermination de la présence et la numération de C. perfringens dans un échantillon, comprenant les étapes suivantes:
- l'ADN de l'échantillon est isolé,
- les parties des gènes de C. perfringens codant respectivement pour la lécithinase et l'entérotoxine sont simultanément amplifiées par polymérisation en chaîne à l'aide d'amorces spécifiques, correspondant respectivement à:
• celles définies dans l'une des revendications 1 à 3, pour l'amplification du gène de l'entérotoxine, et
• une partie de la séquence comprise entre les nucléotides 1350 et 1850 et préférentiellement entre les nucléotides 1350 et 1470 ou entre les nucléotides 1650 et 1850 du gène de la lécithinase, qui est capable d'amplifier une partie dudit gène, en coopérant avec une autre amorce ayant des caractéristiques similaires et une polarité inversée, pour l'amplification d'au moins une partie du gène de la lécithinase, et
- les produits d'amplification sont déterminés à l'aide de procédés connus.

7. Procédé selon la revendication 6, **caractérisé en ce que** les amorces pour l'amplification d'au moins une partie du gène de la lécithinase comprennent presque de 10 à 30 nucléotides.

8. Procédé selon l'une des revendications 6 et 7, **caractérisé en ce que** les amorces pour l'amplification d'au moins une partie du gène de la lécithinase comprennent l'une des séquences suivantes:

SEQ ID1 (PL3) AAG TTA CCT TTG CTG CAT AAT CCC

SEQ ID2 (PL7) ATA GAT ACT CCA TAT CAT CCT GCT

SEQ ID3 (Plc) TCA AAA GAA TAT GCA AGA GGT

SEQ ID4 (PL1) TTC TAT CTT GGA GAGG CTATGCAC

SEQ ID5 (PL4) GCTACTAGTTCTTTTACATTCTTTCC

9. Procédé selon l'une des revendications 5 à 8, dans lequel l'ADN est isolé à partir d'un échantillon alimentaire selon les étapes suivantes:
- l'échantillon est incubé dans un milieu permettant la croissance de C. perfringens,
- les bactéries sont séparées des particules alimentaires par centrifugation,
- les bactéries sont mises en contact avec une résine qui les lyse, et
- l'ADN est séparé des fragments bactériens.
